# EUROPÄISCHE PATENTANMELDUNG

(11) **EP 2 873 381 A1**
(43) Veröffentlichungstag der Anmeldung: **20.05.2015**
(21) Anmeldenummer: 14003803.5
(22) Anmeldetag: 12.11.2014
(51) Int. Cl.: A61B 17/28

(54) **Zangenartiges medizinisches Instrument**

(30) Priorität: 14.11.2013 DE 202013010321 U
(71) Anmelder: Innovations Medical GmbH, 78532 Tuttlingen (DE)
(72) Erfinder: Kreidler, Winfried, 78532 Tuttlingen (DE); Kreidler, Jochen, 78532 Tuttlingen (DE)
(74) Vertreter: Binner, Bernhard

(57) **Zusammenfassung**

Die Erfindung betrifft ein Medizinisches Instrument (1) bestehend aus einem ersten eine Führungsplatte (12) aufweisenden Handhebel (2) und einem zweiten eine Lagerplatte (13) aufweisenden Handhebel (3), welche über einen im Bereich der Führungsplatte (2) und der Lagerplatte (3) angeordneten und mit einem radial erweiterten Kopfteil (26) versehenen Lagerzapfen (6) schwenkbar miteinander verbunden sind, wobei die Lagerplatte (13) in ihren längseitigen Randbereichen jeweils eine Aufnahmenut (17, 18) bildet, in welche die Führungsplatte (12) in ihrer Betriebsstellung eingreift. Zur Verbesserung der Einigung ist erfindungsgemäß vorgesehen, dass die Lagerplatte (13) zwischen den Aufnahmennuten (17, 18) eine sich über die komplette Breite der Lagerplatte (13) erstreckende Vertiefung (14) aufweist und, dass die Führungsplatte (12) aus ihrer Betriebsstellung in eine Reinigungsstellung relativ zur Lagerplatte (13) drehbar ist, in welcher die Führungsplatte (12) mit der Vertiefung (14) in Überdeckung gelangt und, dass im Bereich der Vertiefung (14) zwischen der Führungsplatte (12) und der Lagerplatte (13) ein Distanzelement (39) vorgesehen ist, dessen Höhe (h) in Richtung (15) des Lagerzapfens (6) gleich groß oder kleiner ist als die Tiefe (**T**) der Vertiefung (14).

## Beschreibung

Die Erfindung betrifft ein medizinisches Instrument bestehend aus einem ersten, eine Führungsplatte aufweisenden Handhebel und einem zweiten, eine Lagerplatte aufweisenden Handhebel, welche über einen im Bereich der Führungsplatte und der Lagerplatte angeordneten und mit einem radial erweiterten Kopfteil versehenen Lagerzapfen schwenkbar miteinander verbunden sind, wobei die Lagerplatte in ihren längseitigen Randbereichen jeweils eine Aufnahmenut bildet, in welche die Führungsplatte in ihrer Betriebsstellung eingreift.

Zangenartige oder scherenartige, medizinische Instrumente mit einem ersten Handhebel, der eine Führungsplatte aufweist und einem zweiten Handhebel, welcher eine Lagerplatte aufweist, sind schon seit längerem bekannt. Hierzu sei beispielhaft auf die DE 20 2011 107 977 U1 sowie auf die DE 20 2010 010 843 U1 verwiesen.

Bei den Gegenständen dieser beiden Druckschriften geht es unter anderem auch darum, ein solches medizinisches Instrument oder auch Handwerkzeug reinigen zu können, ohne dass die beiden Handhebel voneinander getrennt werden müssen, das heißt, ohne dass das Instrument bzw. Handwerkzeug zerlegt werden muss.

Beim Gegenstand der DE 20 2010 010 843 U1 weisen die beiden Handhebel jeweils ein Werkzeugteil und ein Griffteil auf. Zwischen dem Werkzeugteil und dem Griffteil ist jeweils ein "plattenartiger" Mittelabschnitt vorgesehen, welcher auch als Führungsplatte und Lagerplatte bezeichnet werden kann.

Im Bereich der Lagerplatte ist - im Wesentlichen mittig - ein Lagerzapfen vorgesehen, welcher ein radial erweitertes Kopfteil aufweist. Die Lagerplatte weist eine erheblich geringere Dicke auf als das Werkzeugteil und als das Griffteil. Dabei ist die Lagerplatte in Richtung des Lagerzapfens versetzt zum Werkzeugteil und zum Griffteil angeordnet, so dass das Werkzeugteil und das Griffteil in Richtung des Lagerzapfens "nach oben" über die Lagerplatte vorsteht.

Um im normalen Betriebszustand eine Verstellung der Führungsplatte mit ihrem Werkzeugteil und ihrem Griffteil in Richtung des Lagerzapfens zu verhindern, sind in den zum Werkzeugteil und zum Griffteil hin liegenden Endbereichen der Lagerplatte, im Übergangsbereich zum Werkzeugteil und im Übergangsbereich zum Griffteil parallel zur Lagerplatte verlaufende Aufnahmenuten vorgesehen, in welche die Führungsplatte im normalen Betriebszustand eingreift. Damit liegt die Führungsplatte mit ihrer lagerplattenseitigen, ebenen Kontaktfläche flächig auf der führungsplattenseitigen "Gleitfläche" der Lagerplatte auf.

Aufgrund dieser besonderen Ausgestaltung sind die beiden Handhebel im normalen Betriebszustand in Richtung des Lagerzapfens nicht axial verstellbar. Die axiale Länge des Lagerzapfens ist beim Gegenstand der DE 20 2010 010 843 U1 derart gewählt, dass dessen radial erweitertes Kopfteil im normalen Betriebszustand einen Abstand "nach außen" zur Führungsplatte aufweist, welcher wenigstens der doppelten Dicke der Führungsplatte entspricht.

Wird nun der Handhebel mit seiner Führungsplatte um den Lagerzapfen gedreht, so gelangt die Führungsplatte nach einem vorbestimmten Drehwinkel mit den Aufnahmenuten der Lagerplatte außer Eingriff, so dass die Führungsplatte entlang des Lagerzapfens verstellt werden kann. Der axiale Stellweg ist dabei durch das radial erweiterte Kopfteil begrenzt. Aufgrund dieser Ausgestaltung werden somit die Kontaktfläche der Führungsplatte und die Gleitfläche der Lagerplatte frei zugänglich, so dass das Handwerkzeug bzw. medizinische Instrument nach der DE 20 2010 010 843 U1 mit einer Reinigungsflüssigkeit gereinigt werden kann.

Nachteilig bei dieser Konstruktion ist, dass die Länge des Lagerzapfens derart gewählt ist, dass die Führungsplatte nach vollständiger Axialverstellung und bei anschließender weiterer Schwenkbewegung der Führungsplatte relativ zum Handhebel mit der Lagerplatte außenseitig im Bereich des Werkzeugteils und im Bereich des Griffteils flächig zur Anlage kommen kann. Somit wird hier bei unsachgemäßer Handhabung zur Reinigung, zumindest ein Teil der Kontaktfläche der Führungsplatte wieder abgedeckt, so dass eine sichere Reinigung nicht gewährleistet werden kann. Des Weiteren ist nicht gewährleistet, dass die Führungsplatte in der Reinigungsstellung unbeabsichtigt in axialer Richtung des Lagerzapfens zurückverstellt wird und die Kontaktfläche der Führungsplatte wieder mit der Gleitfläche lagerplatte flächig zur Anlage gelangt. Dies kann beispielsweise beim Verbringen der Instrumentes in eine entsprechende Reinigungsanlage unbeabsichtigt geschehen.

Da eine solche neuerliche, flächige Anlage der Kontaktfläche und der Gleitfläche der Führungsplatte und der Lageplatte nicht ausgeschlossen werden kann, bedarf es einer großen Aufmerksamkeit der Bedienungsperson, um diese dargestellten Relativpositionen der Führungsplatte zur Lagerplatte und zum Lagerzapfen sicher zu vermeiden.

Beim Gegenstand der Druckschrift DE 20 2011 107 977 U1 sind die Führungsplatte und die Lagerplatte etwas anders ausgestaltet. Hier weist die Lagerplatte keine Aufnahmenuten im Übergangsbereich des Werkzeugteils zur Lagerplatte bzw. im Übergangsbereich des Griffteils zur Lagerplatte auf.

Bei dieser Konstruktion nach der DE 20 2011 107 977 U1 ist in der Führungsplatte ein langlochartiger Durchbruch vorgesehen, welcher in seinem einen Endbereich eine radial erweiterte Einsenkung mit geringer Tiefe aufweist. In seinem zweiten Endbereich ist dieser langlochartige Durchbruch einer zweiten, radial erweiterten Einsenkung versehen, deren Tiefe größer ausgebildet ist als die Tiefe der ersten Einsenkung im ersten Endbereich des langlochartigen Durchbruches. Die Durchmesser der beiden radial erweiterten Einsenkungen ist auf den Durchmesser des radial erweiterten Kopfteils des Lagerzapfens abgestimmt, so dass das Kopfteil des Lagerzapfens wahlweise mit einer der Einsenkungen formschlüssig und drehbar in Eingriff bringbar ist.

Der Lagerzapfen ist auch bei dieser Konstruktion wiederum in der Lagerplatte zentral und feststehend angeordnet.

Um nun das Kopfteil des Lagerzapfens mit der ersten Einsenkung geringerer Tiefe außer Eingriff bringen zu können, weist die Lagerplatte auf Ihrer zur Führungsplatte hin liegenden Gleitfläche eine quer zur Lagerplatte verlaufende Quernut auf. Die Breite dieser Quernut ist wenigstens so groß gewählt wie die Breite der Führungsplatte.

Wird der Handhebel mit der Führungsplatte relativ zur Lagerplatte um den Lagerzapfen soweit gedreht, bis die Führungsplatte auf die Quernut ausgerichtet ist, so kann die Führungsplatte mit ihrem langlochartigen Durchbruch entlang des Lagerzapfens in diese Quernut axial hinein verstellt werden. Dabei gelangt die erste Einsenkung geringerer Tiefe mit dem radial erweiterten Kopfteil des Lagerzapfens außer Eingriff. Anschließend kann die Führungsplatte mit ihrem langlochartigen Durchbruch quer zum Lagerzapfen verstellt werden, bis der Durchbruch mit seiner zweiten radial erweiterten Einsenkung größerer Tiefe mit dem radial erweiterten Kopfteil des Lagerzapfens in Überdeckung gelangt. Durch anschließende Verstellung der Führungsplatte in Richtung des Kopfteils gelangt das Kopfteil mit der zweiten Einsenkung größerer Tiefe in Eingriff. Da die tiefe der zweiten Einsenkung größer ist als die Tiefe der erste Einsenkung weist in dieser "Reinigungsposition" die Führungsplatte zur Lagerplatte einen vordefinierten Abstand auf. Damit ist unabhängig von einer nachfolgenden Veränderung der Winkelstellung der Führungsplatte zur Lagerplatte bzw. der beiden Handhebel zueinander sichergestellt, dass die beiden Kontaktfläche der Führungsplatte und die Gleitfläche der Lagerplatte stets einen Abstand voneinander aufweisen, sofern die Führungsplatte nicht unbeabsichtigt wieder in Richtung des Lagerzapfens axial auf die Lagerplatte zu vertellt wird.

Um eine solche unbeabsichtigte axiale Verstellung der Führungsplatte zur Lagerplatte zu verhindern und dieser Abstand der Führungsplatte zur Lagerplatte in der oben beschriebenen "Reinigungsstellung" erhalten bleibt, kann im Umgebungsbereich des Lagerzapfens eine Tellerfeder vorgesehen sein, durch welche dieser Abstand federelastisch gehalten wird.

Nachteilig bei dieser Konstruktion ist, dass entgegen der Federkraft dieser Tellerfeder das Kopfteil des Lagerzapfens zur Einstellung der Reinigungsposition nur durch Krafteinwirkung mit der ersten radial erweiterten Einsenkung außer Eingriff gebracht werden kann. Umgekehrt ist auch dies, bei der Rückstellung in die normale Betriebsstellung notwendig. Je nach Stärke der Tellerfeder kann hier der Kraftaufwand relativ groß sein, so dass die Handhabung dieses medizinischen Instrumentes in Bezug auf die Verstellung der beiden Handhebel aus der normalen Betriebstellung in die Reinigungsstellung und umgekehrt recht aufwändig ist. Des Weiteren dient bei dieser Konstruktion das Kopfteil des Lagerzapfens als Drehlager, wozu das Kopfteil unterseitig eine umlaufende Kegelfläche aufweist, welche wahlweise in entsprechende, teilweise umlaufende Kegelflächen der beiden Einsenkungen in der Betriebsstellung bzw. in der Reinigungsstellung eingreifen. Somit kann auch hier, insbesondere bei größer auftretenden Betätigungskräften an den Handhebeln, eine sichere Lagerung der Führungsplatte an der Lagerplatte, insbesondere im normalen Betriebszustand, nicht gewährleistet werden.

Demgemäß liegt der Erfindung die Aufgabe zugrunde, ein zangenartige medizinisches Instrument derart zu verbessern, dass eine Reinigung sicher durchführbar ist, ohne dass das Instrument zerlegt werden muss.

Die Aufgabe wird erfindungsgemäß zusammen mit den Merkmalen des Oberbegriffes des Anspruches 1 dadurch gelöst, dass die Lagerplatte zwischen den Aufnahmennuten eine sich über die komplette Breite der Lagerplatte erstreckende Vertiefung aufweist und, dass die Führungsplatte aus ihrer Betriebsstellung in eine Reinigungsstellung relativ zur Lagerplatte drehbar ist, in welcher die Führungsplatte mit der Vertiefung in Überdeckung gelangt und, dass im Bereich der Vertiefung zwischen der Führungsplatte und der Lagerplatte ein Distanzelement vorgesehen ist, dessen Höhe in Richtung des Lagerzapfens gleich groß oder kleiner ist als die Tiefe der Vertiefung.

Durch die erfindungsgemäße Ausgestaltung wird ein zangenartiges, medizinisches Handwerkzeug zur Verfügung gestellt, dessen Reinigungsposition sicher und einfach einstellbar ist. Des Weiteren wird auch eine eindeutige Drehlagerung der Führungsplatte relativ zur Lagerplatte durch den Lagerzapfen erreicht. Aufgrund seines radial erweiterten Kopfteils wird ebenfalls erreicht, dass die beiden Handhebel über ihre Führungsplatte und ihre Lagerplatte unverlierbar miteinander in Verbindung stehen, unabhängig ob diese sich bezüglich ihrer Winkelstellung in der Betriebsstellung oder in der Reinigungsstellung befinden.

Weitere vorteilhafte Ausgestaltungen der Erfindung sind den weiteren Unteransprüchen entnehmbar.

So kann gemäß Anspruch 2 vorgesehen sein, dass die Vertiefung in Längsrichtung der Lagerplatte beidseitig durch jeweils einen quer zur Lagerplatte verlaufenden Abschnitt und einen sich daran anschließenden schräg zur Längsmittelebene der Lagerplatte verlaufenden Abschnitt begrenzt ist. Die schräg verlaufenden Abschnitte können dabei unter einem Winkel von ca. 60° zu einer Längsmittelebene des medizinischen Instruments verlaufend angeordnet sein. Auf Grund dieser schräg verlaufenden Abschnitte ist die Reinigungsstellung nach einer relativen Drehung des ersten Handhebels zum zweiten Handhebels um ca. 60° erreicht. Dabei bleibt diese Reinigungsstellung erhalten, wenn der erst Handhebel zum zweiten Handhebel um weitere 30° gedreht wird.

Weiter kann gemäß Anspruch 3 vorgesehen sein, dass das erweiterte Kopfteil des Lagerzapfens im axialen Bereich einer äußeren Oberfläche der Führungsplatte angeordnet ist und, dass die zur Führungsplatte hin liegende "untere" Begrenzungsfläche des radial erweiterten Kopfteils in der Betriebsstellung der beiden Handhebel bzw. der Führungsplatte zur Lagerplatte einen minimalen Abstand zur Oberfläche der Führungsplatte aufweist. Durch diese Ausgestaltung wird erreicht, dass das Kopfteil nach Verstellung aus der Betriebsstellung in die Reinigungsstellung sicher mit Reinigungsflüssigkeit umspült werden kann.

Gemäß Anspruch 4 kann vorgesehen sein, dass die Führungsplatte in ihrer Oberfläche im Umgebungsbereich des Kopfteils eine Einsenkung aufweist, deren Durchmesser größer ist, als der Durchmesser des radial erweiterten Kopfteils. auch durch diese Ausgestaltung wird sichergestellt, dass insbesondere das Kopfteil sicher mit Reinigungsflüssigkeit umspült werden kann. Des Weiteren ergibt sich hierdurch auch die Möglichkeit, die beiden Handhebel in der Reinigungsstellung axial in Richtung des Lagerzapfen gegeneinander zu verstellen, um den Abstand der einander zugewandten Flächen der Führungsplatte und der Lageplatte zu vergrößern, so dass auch das Distanzelement vollständig "frei" liegt und dementsprechend mit Reinigungsflüssigkeit umspült werden kann.

Gemäß Anspruch 5 kann die axiale Länge des Lagerzapfens derart bemessen sein, dass die untere, zur Führungsplatte hin liegende Begrenzungsfläche des radial erweiterten Kopfteils des Lagerzapfens einen minimalen Abstand zur "inneren" Kreisringfläche der Einsenkung aufweist. Durch diese Ausgestaltung ist das Kopfteil ebenfalls sicher umspülbar.

Gemäß Anspruch 6 kann vorgesehen sein, dass das Distanzelement als umlaufender Ringsteg ausgebildet ist, welcher koaxial zum Lagerzapfen zwischen der Führungsplatte und der Vertiefung der Lagerplatte angeordnet ist. Durch diese Ausgestaltung ergibt sich eine äußerst geringe Anlagefläche des Distanzelementes an der Lagerplatte bzw. der Führungsplatte. Damit wird erreicht, dass bei Anlage des Distanzelementes an der Führungs- und/oder Lagerplatte nur unwesentlich kleine Bereiche der Oberflächen der Führungs- und/oder Lagerplatte für die Reinigungsflüssigkeit nicht zugänglich sind.

Weiter kann gemäß Anspruch 7 vorgesehen sein, dass das Distanzelement wahlweise als separates Bauteil oder als einstückiger Bestandteil der Führungsplatte oder der Lagerplatte ausgebildet ist. Ist das Distanzelement als einstückiger Bestandteil der Führungsplatte oder der lagerplatte ausgebildet, so kann das Distanzelement auch mehrteilig ausgebildet sein. Beispielweise können zwei oder mehrere Stegelement als Distanzelement zwischen der Führungsplatte und der Lagerplatte vorgesehen sein. Auch hierdurch ergeben sich äußerst kleine Anlagefläche der einzelnen Elemente des Distanzelementes an der jeweils gegenüber liegenden Fläche der Führungsplatte oder der Lagerplatte, welche für eine optimale Reinigung des Instrumentes vernachlässigbar klein sind.

Anhand der Zeichnung wird beispielhaft anhand einer schematisch dargestellten Ausführungsvariante eines zangenartigen medizinischen Instrumentes, die Erfindung näher erläutert. Es zeigt:
- Fig. 1: eine perspektivische Darstellung eines zangenartigen medizinischen Instrumentes mit zwei Handhebeln, welche jeweils ein Werkzeugteil und ein Griffteil aufweisen, zwischen welchen eine Führungsplatte bzw. eine Lagerplatte vorgesehen ist, welche über einen Lagerzapfen drehbar miteinander in Verbindung stehen;
- Fig. 2: eine vergrößerte, teilweise perspektivische Explosionsdarstellung des Lagerzapfens zusammen mit einer perspektivischen Unteransicht des ersten Handhebels mit seiner Führungsplatte sowie einer teilweise perspektivischen Oberansicht des Handhebels im Bereich seiner Lagerplatte;
- Fig. 3: eine teilweise Schnittdarstellung III-III aus Fig. 1 des medizinischen Instrumentes im Bereich der Führungsplatte und der Lagerplatte;
- Fig. 4: eine perspektivische Darstellung des medizinischen Instrumentes aus Fig. 1 in einer geöffneten Reinigungsstellung seiner beiden Handhebel;
- Fig. 5: eine Schnittdarstellung V-V aus Fig. 4 im Bereich der Führungsplatte und der Lagerplatte.

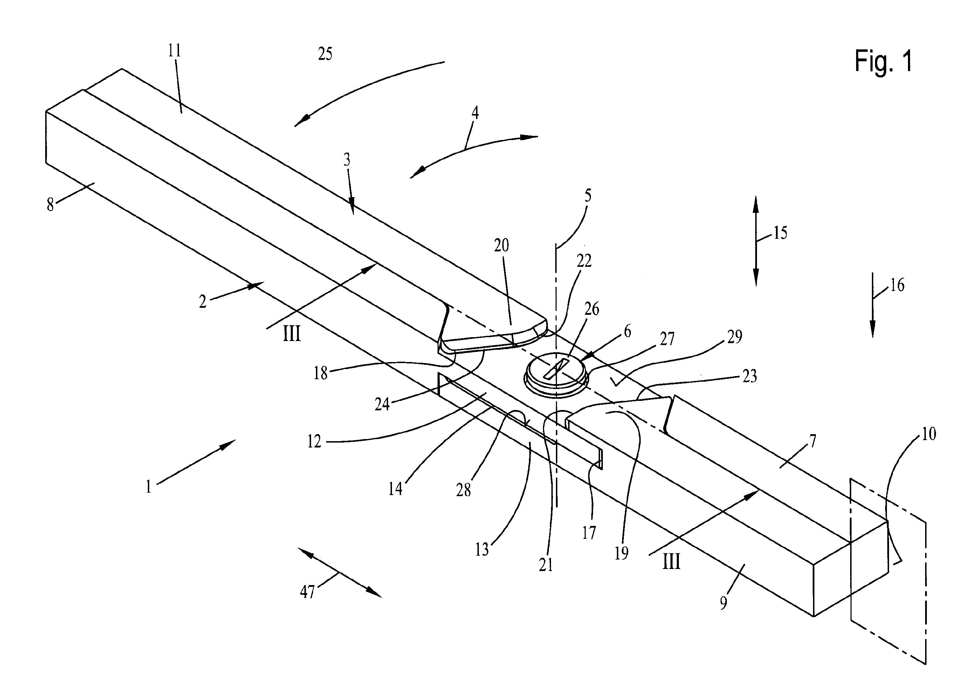

Fig. 1 zeigt eine perspektivische Darstellung eines zangenartigen medizinischen Instruments 1, welches aus einem ersten Handhebel 2 und einem zweiten Handhebel 3 besteht. Der erste Handhebel 2 ist relativ zum zweiten Handhebel 3 in Richtung des Doppelpfeils 4 um eine Drehachse 5 drehbar gelagert. Zur drehbaren Lagerung ist bei der dargestellten Ausführungsvariante ein entsprechender Lagerzapfen 6 vorgesehen.

Des Weiteren ist aus Fig. 1 erkennbar, dass der erste Handhebel 2 ein erstes Werkzeugteil 7 sowie ein Griffteil 8 aufweist, welche sich bezüglich der Drehachse 5 diametral gegenüberliegen.

Der zweite Handhebel 3 weist ebenfalls ein Werkzeugteil 9 auf, welches bezüglich einer Längsmittelebene 10 dem ersten Werkzeugteil 7 des ersten Handhebels 2 gegenüberliegt. Diesem zweiten Werkzeugteil 9 bezüglich der Drehachse 5 diametral gegenüberliegend weist auch der zweite Handhebel 3 ein Griffteil 11 auf. Dabei sei an dieser Stelle angemerkt, dass sowohl die Werkzeugteile 7 und 9 als auch die Griffteile 8 und 11 in der Zeichnung lediglich schematisch dargestellt sind und in ihrer Ausgestaltung annähernd beliebig ausgebildet sein können, wobei hierzu auf die eingangs genannten Druckschriften DE 20 2010 010 843 U1 sowie die DE 20 2011 107 977 U1 beispielhaft hingewiesen wird.

Der erste Handhebel 2 bildet zwischen seinem Werkzeugteil 7 und seinem Griffteil 8 eine Führungsplatte 12, mit welcher das erste Werkzeugteil 7 auf dem Lagerzapfen 6 drehbar gelagert ist.

Das zweite Werkzeugteil 9 bildet zwischen seinem Werkzeugteil 9 und seinem Griffteil 11 eine Lagerplatte 13, in welcher der Lagerzapfen 6 feststehend eingesetzt ist.

Weiter ist erkennbar, dass die Lagerplatte 13 zur Führungsplatte 12 hin eine Vertiefung 14 aufweist, welche sich über die vollständige Breite der Lagerplatte 13 erstreckt. Damit weist die Lagerplatte 13 in diesem Bereich einen "axialen" Abstand in Richtung des Doppelpfeils 15 der Drehachse 5 zur Führungsplatte 12 auf.

Des Weiteren ist erkennbar, dass die Lagerplatte 13 vertikal in Richtung des Pfeils 16 versetzt sowohl zum Werkzeugteil 9 als auch zum Griffteil 11 zwischen diesen beiden Teilen angeordnet ist. Die Führungsplatte 12 des ersten Handhebels 2 steht in dem in Fig. 1 dargestellten geschlossenen Zustand des medizinischen Instrumentes 1 mit der Lagerplatte 13 im Umgebungsbereich der Vertiefung 14 flächig in Kontakt. Die Lagerplatte 13 weist einerseits zu ihrem Werkzeugteil 9 und andererseits zu ihrem Griffteil 11 hin jeweils eine Aufnahmenut 17 bzw. 18 auf, in welche die Führungsplatte 12 in dem dargestellten geschlossenen Zustand passend eingreift.

Die beiden Aufnahmenuten 17 und 18 werden dabei oberseitig jeweils durch einen Anschlagsteg 19 bzw. 20 begrenzt. Jeder der Anschlagstege 19 bzw. 20 bildet einen quer zur Längserstreckung des Doppelpfeils 46 des zweiten Handhebels 3 verlaufende Begrenzungskante 21 bzw. 22, an welche sich jeweils ein schräg zur Längserstreckung verlaufender Kantenabschnitt 23 anschließt.

Wird der erste Handhebel 2 mit seiner Führungsplatte 12 in Richtung des Pfeiles 25 gedreht, so öffnen sich sowohl die beiden Werkzeugteile 7 und 9 als auch die beiden Griffteile 8 und 11. Nach einem gewissen, vorbestimmten relativen Drehwinkel des ersten Handhebels 2 mit seiner Führungsplatte 12 in Richtung des Pfeiles 25 gelangt die Führungsplatte 12 mit den beiden Aufnahmenuten 17 und 18 der Lagerplatte 13 außer Eingriff. Bei der dargestellten Ausführungsvariante liegt dieser Drehwinkel bei etwa 60°, was durch den schrägen Verlauf der beiden Kantenabschnitte 23 und 24 definiert ist.

Des Weiteren ist aus Fig. 1 andeutungsweise erkennbar, dass der Lagerzapfen 6 ein radial erweitertes Kopfteil 26 aufweist. Im Bereich dieses Kopfteiles 26 bildet die Führungsplatte 12 eine umlaufende Einsenkung 27. Diese Einsenkung 27 ist in ihrem Durchmesser größer ausgebildet als das Kopfteil 26 des Lagerzapfens 6.

Bei der dargestellten Ausführungsvariante ist die Länge des Lagerzapfens 6 derart ausgebildet, dass das Kopfteil 26 mit seiner unteren Begrenzungsfläche 28 etwa im Bereich der Ebene der oberen Begrenzungsflächen 29 der Führungsplatte 12 liegt. Dadurch ist unter anderem, sofern keine Axialverstellung der Führungsplatte 12 in der geöffnetem Reinigungsstellung relativ zum Lagerzapfen 6 erfolgt, die untere Begrenzungsfläche 28 des Kopfteils 26 des Lagerzapfens 6 für entsprechende Reinigungsflüssigkeit frei zugänglich.

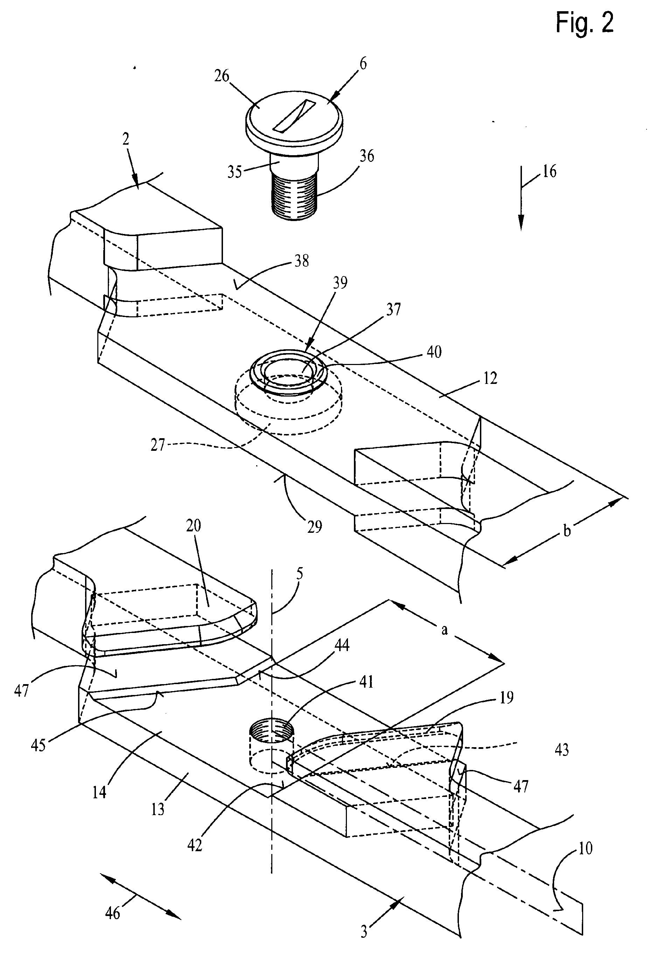

Fig. 2 zeigt eine Explosionsdarstellung der verkürzt dargestellten Handhebel 2 und 3 zusammen mit dem Lagerzapfen 6. Dabei ist der erste Handhebel 2 sowie der zweite Handhebel 3 lediglich im Bereich der Führungsplatte 12 bzw. der Lagerplatte 13 dargestellt.

Der erste Handhebel 2 ist wiederum mit seiner Führungsplatte 12 von seiner Unterseite dargestellt, während der Handhebel 3 mit seiner Lagerplatte in der gleichen Position von der Oberseite her sichtbar dargestellt ist, wie dies in Fig. 1 erkennbar ist.

Aus Fig. 2 ist ersichtlich, dass das Kopfteil 26 des Lagerzapfens 6 radial erweitert ausgebildet ist. Dementsprechend bildet der Lagerzapfen 6 "unterhalb" des Kopfteils 26 einen radial verjüngt ausgebildeten Lagerzylinder 35, an welchen sich nach unten hin ein nochmals radial verjüngt ausgebildeter Gewindeabschnitt 36 anschließt. Der Lagerzapfen 6 ist mit seinem Gewindeabschnitt 36 und seinem Lagerzylinder 35 durch eine Lagerbohrung 37 der Führungsplatte 12 hindurch steckbar. Da die Führungsplatte 12 von der Unterseite her dargestellt ist, erfolgt dieses Hindurchstecken von der in Fig. 2 nun "unten" angeordneten oberen Begrenzungsfläche 29 der Führungsplatte 12.

Dabei ist aus Fig. 2 in gestrichelten Linien die bereits oben beschriebene Einsenkung 27 erkennbar, in welcher sich die Lagerbohrung 37 fortsetzt.

Weiter ist aus Fig. 2 erkennbar, dass im Umgebungsbereich der Lagerbohrung 37 im Bereich der Kontaktfläche 38 der Führungsplatte 12, ein Distanzelement in Form eines Ringsteges 39 vorgesehen ist, welcher eine schmale Stützfläche 40 bildet. Über diese kreisringförmige Stützfläche 40 des Ringsteges 39 stützt sich die Führungsplatte 12 axial im "geöffneten" Reinigungszustand "oberseitig" auf der Lagerplatte 13 ab. Damit wird sichergestellt, dass die Führungsplatte 12 in der geöffneten Reinigungsstellung nicht unbeabsichtigt mit der Vertiefung 14 der Lagerplatte 13 in Kontakt gelangen kann und somit beide Teil zu Reinigungszwecken mit einer Reinigungsflüssigkeit beaufschlagbar sind.

Die Lagerplatte 13 weist zur Aufnahme des Gewindeabschnittes 36 des Lagerzapfens 6 zentral ein entsprechendes Innengewinde 41 auf, in welches der Lagerzapfen 6 feststehend einschraubbar ist.

Des Weiteren ist aus Fig. 2 ersichtlich, dass die Kontur der "versenkt" in der Lagerplatte 13 angeordneten Vertiefung 14 etwa der Kontur der beiden Anschlagstege 19 und 20 entspricht. Dementsprechend weist die Begrenzung der Vertiefung 14 im Bereich des Anschlagsteges 19 einen quer verlaufenden Abschnitt 42 sowie einen schräg verlaufenden Abschnitt 43 als Begrenzungskante der Vertiefung 14 auf. Die dem Anschlagsteg 20 zugeordnete Begrenzung der Vertiefung 14 weist zur "Rückseite" der Lagerplatte 13 hin einen quer verlaufenden Abschnitt 44 und zur "Frontseite" der Lagerplatte 13 hin einen schräg verlaufenden Abschnitt 45 auf. Der Abstand **a** der beiden quer verlaufenden Abschnitte 42 und 44 entspricht dabei mindestens der Breite **b** der Führungsplatte 12. Dementsprechend kann die Führungsplatte 12 in Richtung des Pfeils 16 in einer rechtwinklig zur Lagerplatte 13 verstellten Winkelposition mit dem zwischen den beiden Abschnitten 42 und 44 liegenden Bereich der Vertiefung in Überdeckung gebracht werden.

Aufgrund des in Fig. 2 "nach oben" und im zusammengebauten Zustand der Fig. 1 nach unten vorstehenden Ringsteges 39 kann jedoch keine Axialbewegung in Richtung des Pfeils 16 bzw. in Richtung des Lagerzapfens 6 in die Vertiefung 14 hinein erfolgen.

Die beiden sich bezüglich der Längsmittelachse bzw. Drehachse 5 spiegelsymmetrisch gegenüber liegenden, schrägen Abschnitte 43 und 45, verlaufen zur durch die Drehachse 5 hindurch gehenden Längsmittelebene 10 unter einem Schrägungswinkel von etwa 60°. Dieser Schrägungswinkel kann auch andere Werte annehmen.

Dies bedeutet, dass aufgrund der besonderen Ausgestaltung der Begrenzung der Vertiefung 14 durch die Abschnitte 42 und 43 sowie 44 und 45, die Führungsplatte 12 nach einer Drehung in Richtung des Pfeils 25 (Fig. 1) um etwa 60° bereits zwischen diesen beiden Abschnitten 43 und 45 angeordnet ist, so dass keine Berührung mehr zwischen der Kontaktfläche 38 der Führungsplatte 12 und der "Gleitfläche" 47 (Fig. 1) der Lagerplatte 13 stattfinden kann. In einer solchen Schwenklage liegen die einander zugewandten Flächen 38 sowie 47 wie auch die Oberfläche der Vertiefung 14 völlig frei, so dass diese für ein Reinigungsmittel frei zugänglich sind.

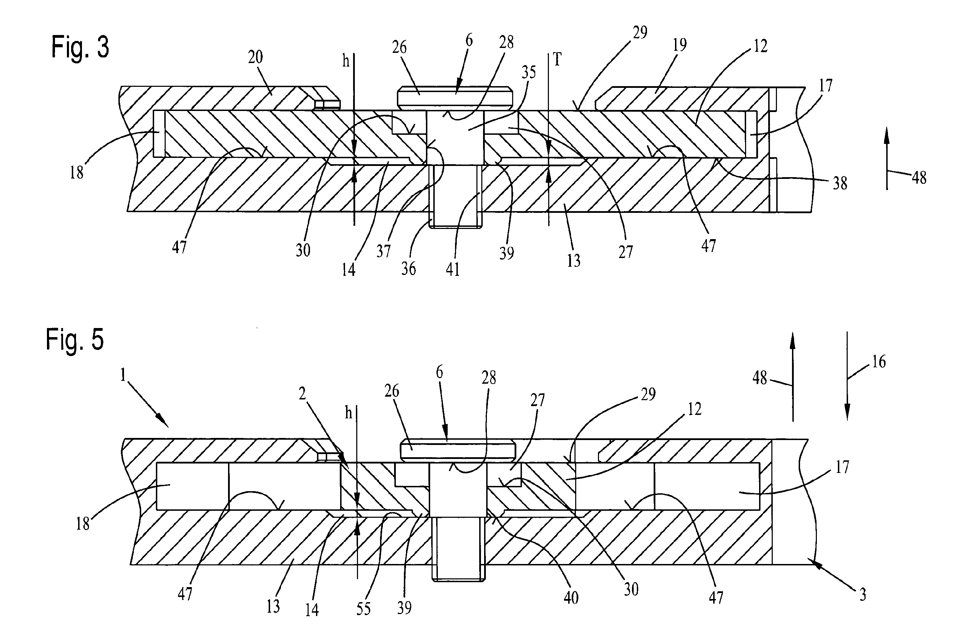

Hierzu zeigt Fig. 3 einen Schnitt III-III aus Fig. 1 im Bereich der Führungsplatte 12 und der Lagerplatte 13. Es ist erkennbar, dass die Führungsplatte 12 in den beiden Aufnahmenuten 17 und 18 der Lagerplatte 13 passen aufgenommen ist und mit ihrer unteren Kontaktfläche 38 flächig auf der Gleitfläche 47 der Lagerplatte 13 anliegt. Zur Verhinderung einer Axialbewegung in Richtung des Pfeiles 48 entlang des Lagerzapfens 6, sind die beiden oberen Anschlagstege 19 und 20 vorgesehen, so dass die Führungsplatte 12 in dem in Fig. 3 dargestellten geschlossenen Betriebszustand, passend in den beiden Aufnahmenuten 17 und 18 aufgenommen wird.

Weiter ist aus Fig. 3 erkennbar, dass die vertikale Höhe **h** des den Lagerzapfen 6 umgebenden Ringsteges 39 etwa identisch ist mit der Tiefe **T** der Vertiefung 14. Dies Höhe **h** kann auch kleiner sein als die Tiefe **T** der Vertiefung 14.

Weiter ist aus der Schnittdarstellung der Fig. 3 erkennbar, dass der radial erweiterte Kopfteil 26 des Lagerzapfens 6 mit seiner unteren Begrenzungsfläche 28 etwa in derselben Ebene liegt wie die obere Begrenzungsfläche 29 der Führungsplatte 12.

Weiter ist aus Fig. 3 erkennbar, dass die Einsenkung 27 der Lagerbohrung 37 einen größeren Durchmesser aufweist als das radial erweiterte Kopfteil 26 des Lagerzapfens 6. In dem in Fig. 3 dargestellten montierten Zustand ist der Lagerzapfen 6 mit seinem Gewindeabschnitt 36 in das Innengewinde 41 der Lagerplatte 13 eingeschraubt. Der Lagerzylinder 35 des Lagerzapfens 6 greift in dieser montierten Position passend in die Lagerbohrung 37 der Führungsplatte 12 ein. Aus der Darstellung der Fig. 3 ist erkennbar, dass aufgrund des größeren Durchmessers der Einsenkung 27 relativ zum radial erweiterten Kopfteil 26 und des axialen Abstandes der unteren Begrenzungsfläche 28 des Kopfteils 26 von der inneren Kreisringfläche 30 der Einsenkung 27, eine Umspülung unterhalb des Kopfteils 26 mit Reinigungsflüssigkeit sicher möglich ist.

Im Weiteren kann auch vorgesehen sein, dass die Länge des Lagerzapfens derart ausgebildet ist, dass die untere Begrenzungsfläche 28 des Kopfteils 26 einen geringen Abstand zur von der Einsenkung 27 gebildeten Kreisringfläche 30 aufweist. Auch hierdurch wird eine Umspülbarkeit in diesem Bereich, insbesondere in der Reinigungsstellung der beiden Handhebel 2 und 3 zueinander, mit Reinigungsflüssigkeit erreicht.

Zur Einstellung der Reinigungsstellung ist bei der dargestellten Ausführungsvariante eine Drehbewegung des ersten Handhebels 2 in Richtung des Pfeiles 25 um wenigstens 60° und höchstens 90° erforderlich.

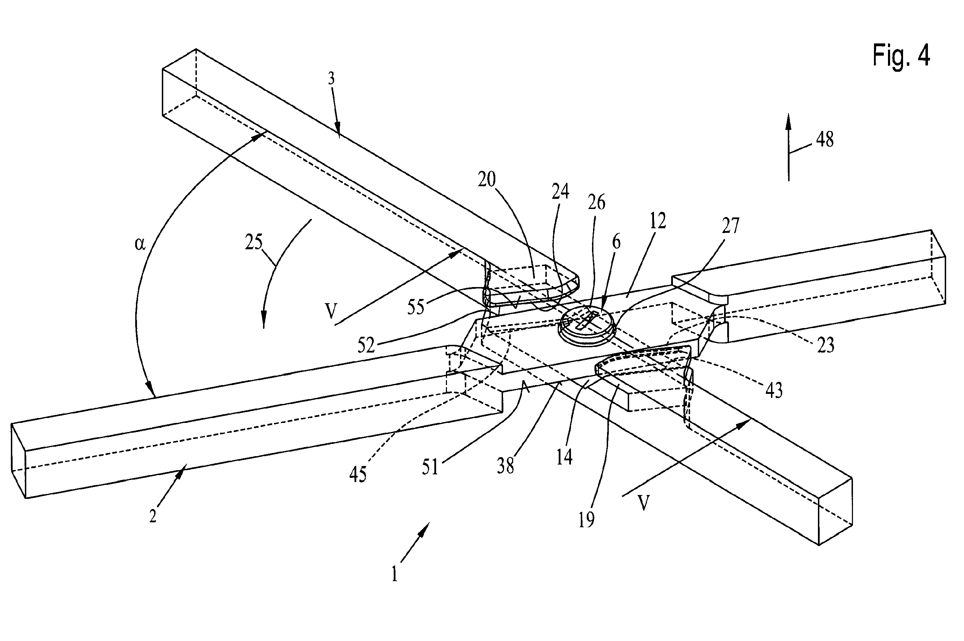

Fig. 4 zeigt hierzu eine perspektivische Darstellung des medizinischen Instrumentes 1 in einer Reinigungsstellung, bei welcher der erste Handhebel 2 relativ zum zweiten Handhebel 3 um einen Drehwinkel α von 60° in Richtung des Pfeils 25 verstellt ist.

Es ist erkennbar, dass in dieser gedrehten Winkelstellung die Führungsplatte 12 mit ihren seitlichen Begrenzungskanten 51 und 52 zwischen den schräg verlaufenden Kantenabschnitten 23 und 24 der beiden Anschlagstege 19 und 20 und somit auch parallel zu den ebenfalls im gleichen Winkel, wie oben bereits erwähnt, verlaufenden Abschnitten 43 und 45 der Vertiefung 14 verläuft. Der Abstand dieser beiden schräg verlaufenden Abschnitte 43 und 45 voneinander ist dabei zumindest minimal größer gewählt als die Breite **b** (Fig. 2) der Führungsplatte 12. In dieser Winkelstellung weist die untere Kontaktfläche 38 einen Abstand von der Oberfläche 55 der Vertiefung 14 auf. Aufgrund des unterseitig im Umgebungsbereich des Lagerzapfens 6 vorgesehenen Ringsteges 39 (in Fig. 4 nicht dargestellt), kann dieser Abstand nicht kleiner werden als die Höhe **h** des Ringsteges 39, so dieser Abstand zu Reinigungszwecken sicher erhalten bleibt.

Aufgrund der Einsenkung 27 im Umgebungsbereich des Kopfteils 26 des Lagerzapfens 6, ist es jedoch möglich, jedoch nicht zwingend erforderlich, den ersten Handhebel 2 mit seiner Führungsplatte 12 in Richtung des Pfeiles 48 zu verstellen.

Fig. 5 zeigt eine Schnittdarstellung V-V des medizinischen Instrumentes 1 aus Fig. 4. Es ist erkennbar, dass die Führungsplatte 12 in der in Fig. 4 dargestellten Winkelstellung der beiden Handhebel 2 und 3 nicht mehr mit den beiden Aufnahmenuten 17 und 18 in Eingriff steht. In dieser Position befindet sich die Führungsplatte 12 voll umfänglich im Bereich der vertikal in Richtung des Pfeiles 16 unterhalb der Gleitflächen 47 liegenden Oberfläche 55 der Vertiefung 14 und weist zu dieser Oberfläche 55 einen entsprechenden Abstand auf, welcher der "axialen" Höhe **h** des Ringsteges 39 entspricht.

Der Ringsteg 39 liegt mit seiner äußerst schmalen Stützfläche 40 auf der Oberfläche 55 auf. Bei normaler Handhabung des Instrumentes 1 und der nunmehr frei liegenden Führungsplatte 12 kann diese sich in Richtung des Pfeiles 48 bewegen, so dass ein Durchspülen der zu reinigenden Teile in der in Fig. 5 im Schnitt dargestellten Reinigungsposition der Führungsplatte 12 relativ zur Lagerplatte 13 optimal erfolgen kann.

Da das Kopfteil 26 des Lagerzapfens 6 einen kleineren Durchmesser aufweist als die Einsenkung 27 und diese Einsenkung 27 nur im Extremfall mit der unteren Begrenzungsfläche 28 des Kopfteils 26 mit ihrer Kreisringsfläche 30 in Berührung kommt, ist eine optimale Reinigung sicher möglich.

Dabei kann die Einsenkung 27 bei Bedarf auch weggelassen werden, wobei die axiale Länge des Lagerzapfens 6 in diesem Falle so zu wählen ist, dass die Unterseite 28 des Kopfteils 26 einen zumindest minimalen Abstand von der oberen Begrenzungsfläche 29 der Führungsplatte 12 aufweist (in der Zeichnung nicht dargestellt).

Somit ist zusammenfassend festzustellen, dass durch die erfindungsgemäße Ausgestaltung des zangenartigen, medizinischen Instrumentes 1 eine optimale Reinigung bei gleichzeitig äußerst einfacher Handhabung, insbesondere betreffend der Einstellung der Reinigungsstellung der beiden Handhebel 2 und 3 zueinander, ermöglicht wird.

Hierzu sei angemerkt, dass insbesondere der als Distanzelement eingesetzte Ringsteg 39 einerseits einstückiger Bestandteil der Führungsplatte 12 aber auch wahlweise der Lagerplatte 13 sein kann. Weiter kann dieser Ringsteg 39 als separates Bauteil ausgebildet sein, welches "lose" zwischen die Führungsplatte 12 und die Lagerplatte 13 eingesetzt ist.

Auch sind bezüglich des Distanzelementes andere Ausgestaltungen vorstellbar. Beispielsweise können hier einzelne Stegelemente, beispielsweise auf der Oberfläche 55 der Vertiefung 14 der Lagerplatte 13 oder auch auf der Kontaktfläche 38 der Führungsplatte 12 vorgesehen sein.

Es ist hier lediglich notwendig, die Berührungsfläche solcher Distanzelemente möglichst klein zu halten, so dass insbesondere die Kontaktfläche 38 sowie die Gleitfläche 47 in der Reinigungsstellung der beiden Handhebel 2 und 3 möglichst vollflächig frei zugänglich sind.

Da, wie zum dargestellten Ausführungsbeispiel ausgeführt, eine relative Verstellung der Führungsplatte 12 zur Lagerplatte 13 in Richtung des Pfeiles 48 entlang des Lagerzapfens aufgrund der vorgesehenen Einsenkung 27 möglich ist, wird sich in der Praxis nach Einstellung der Reinigungsstellung der beiden Handhebel 2 und 3 zueinander, im Wesentlichen stets eine mittlere Stellung der Führungsplatte 12 relativ zum Lagerzylinder 35 des Lagerzapfens 6 ergeben, so dass eine optimale Reinigung des medizinischen Instrumentes sicher möglich ist.

Aufgrund der besonderen Formgestaltung der Vertiefung 14 mit seinen Begrenzungen 42, 43, 44 und 45, ist des Weiteren ein Winkelbereich von insgesamt 30° als Reinigungsstellung möglich, so dass die Bedienperson nicht speziell darauf achten muss, dass die Führungsplatte 12 präzise auf die Vertiefung 14 auszurichten ist.

Wie bereits oben erwähnt, wird beim vorliegenden Ausführungsbeispiel nach einer Verstellung von 60° der beiden Handhebel 2 und 3 zueinander aus der geschlossenen Betriebsstellung der Fig. 1 in die geöffnete Stellung der Fig. 4, bereits die Reinigungsstellung erreicht. Dabei kann der Handhebel 2 relativ zum Handhebel 3 noch um weitere 30° verstellt werden, so dass der in Fig. 4 eingetragene Stellwinkel α maximal bis zu 90° für die Reinigungsstellung betragen kann. Auch dies ist, insbesondere bezüglich der Handhabung zum Erreichen der Reinigungsstellung der beiden Handhebel 2 und 3 zueinander, äußerst vorteilhaft.

## Patentansprüche

1. Medizinisches Instrument (1) bestehend aus einem ersten eine Führungsplatte (12) aufweisenden Handhebel (2) und einem zweiten eine Lagerplatte (13) aufweisenden Handhebel (3), welche über einen im Bereich der Führungsplatte (2) und der Lagerplatte (3) angeordneten und mit einem radial erweiterten Kopfteil (26) versehenen Lagerzapfen (6) schwenkbar miteinander verbunden sind, wobei die Lagerplatte (13) in ihren längseitigen Randbereichen jeweils eine Aufnahmenut (17, 18) bildet, in welche die Führungsplatte (12) in ihrer Betriebsstellung eingreift,
**dadurch gekennzeichnet,**
**dass** die Lagerplatte (13) zwischen den Aufnahmennuten (17, 18) eine sich über die komplette Breite der Lagerplatte (13) erstreckende Vertiefung (14) aufweist und,
**dass** die Führungsplatte (12) aus ihrer Betriebsstellung in eine Reinigungsstellung relativ zur Lagerplatte (13) drehbar ist, in welcher die Führungsplatte (12) mit der Vertiefung (14) in Überdeckung gelangt und,
**dass** im Bereich der Vertiefung (14) zwischen der Führungsplatte (12) und der Lagerplatte (13) ein Distanzelement (39) vorgesehen ist, dessen Höhe (**h**) in Richtung (15) des Lagerzapfens (6) gleich groß oder kleiner ist als die Tiefe (**T**) der Vertiefung (14).

2. Instrument nach Anspruch 1, **dadurch gekennzeichnet, dass** die Vertiefung (14) in Längsrichtung (46) der Lagerplatte (13) beidseitig durch jeweils einen quer zur Lagerplatte (13) verlaufenden Abschnitt (42, 44) und einen sich daran anschließenden schräg zur Längsmittelebene (10) der Lagerplatte (13) verlaufenden Abschnitt (43, 35) begrenzt ist.

3. Instrument nach Anspruch 1 oder 2, **dadurch gekennzeichnet, dass** das erweiterte Kopfteil (26) des Lagerzapfens (6) im axialen Bereich einer äußeren Oberfläche (29) der Führungsplatte (12) angeordnet ist und, dass die zur Führungsplatte (12) hin liegende "untere" Begrenzungsfläche (28) des radial erweiterten Kopfteils (26) in der Betriebsstellung der beiden Handhebel (2, 3) bzw. der Führungsplatte (12) zur Lagerplatte (13) einen minimalen Abstand zur Oberfläche (29) der Führungsplatte (12) aufweist.

4. Instrument nach einem der Ansprüche 1 oder 2, **dadurch gekennzeichnet, dass** die Führungsplatte (12) in ihrer Oberfläche (29) im Umgebungsbereich des Kopfteils (26) eine Einsenkung (27) aufweist, deren Durchmesser größer ist, als der Durchmesser des radial erweiterten Kopfteils (26).

5. Instrument nach Anspruch 4, **dadurch gekennzeichnet, dass** die axiale Länge des Lagerzapfens (6) derart bemessen ist, dass die untere, zur Führungsplatte (12) hin liegende Begrenzungsfläche (28) des radial erweiterten Kopfteils (26) des Lagerzapfens (6) einen minimalen Abstand zur "inneren" Kreisringfläche (30) der Einsenkung aufweist.

6. Instrument nach einem der Ansprüche 1 bis 5, **dadurch gekennzeichnet, dass** das Distanzelement als umlaufender Ringsteg (39) ausgebildet ist, welcher koaxial zum Lagerzapfen zwischen der Führungsplatte und der Vertiefung (14) der Lagerplatte (13) angeordnet ist.

7. Instrument nach einem der Ansprüche 1 bis 6, **dadurch gekennzeichnet, dass** das Distanzelement (39) wahlweise als separates Bauteil oder als einstückiger Bestandteil der Führungsplatte (12) oder der Lagerplatte (13) ausgebildet ist.
